# NEUE EUROPÄISCHE PATENTSCHRIFT

(11) **EP 0 491 125 B2**
(45) Veröffentlichungstag und Bekanntmachung des Hinweises auf die Entscheidung über den Einspruch: **24.10.2001**
(45) Hinweis auf die Patenterteilung: 17.04.1996
(21) Anmeldenummer: 91117027.2
(22) Anmeldetag: 07.10.1991
(51) Int. Cl.: A61K 39/245, A61K 39/27

(54) **Impfstoffe gegen equine Herpesviren**
Vaccines against equine herpes viruses
Vaccins contre les virus de l'herpès équin

(30) Priorität: 20.10.1990 DE 4033446
(43) Veröffentlichungstag der Anmeldung: 24.06.1992
(73) Patentinhaber: BAYER AG, 51368 Leverkusen (DE)
(72) Erfinder: Thein, Peter, Prof. Dr. Dr.habil, W-8064 Oberzeitlbach (DE); Strube, Walter, Dr., W-5000 Köln 40 (DE); Böttcher, Ernst, W-8000 München (DE)

(56) Entgegenhaltungen:
- EP-A- 0 001 365
- WO-A-90/01546
- VACCINE, vol. 8, no. 5, October 1990, pages 491-496; R.F. COOK et al.: "Protection against lethal equine herpes virus type 1 (subtype 1) infection in hamsters by immune stimulating complexes (ISCOMS) containing the major viral glycoproteins"

## Beschreibung

Die vorliegende Erfindung betrifft Impfstoffe für Pferde gegen Infektionen mit equinen Herpesviren, sowie Komplexe aus Glycoproteinen von equinen Herpesviren und Lektinen.

Die equinen Herpesviren (EHV) sind enzootisch in allen Pferdezuchtgebieten der Welt verbreitet und spielen eine dominierende Rolle innerhalb der Infektionskrankheiten der Pferde. Nach Primärinfektion kann es zu einer lebenslangen Persistenz im infizierten Tier kommen. Bislang konnten bei Pferden insgesamt vier Herpesvirus-Serotypen identifiziert werden.
- EHV-1: (Equines Abortvirus), ein zu den Alphaherpesviren zählender Erreger, vormals als EHV-1 Subtyp 1 (Rhinopneumonitisvirus) bezeichnet,
- EHV-2: (Equines Cytomegalo like virus), ein Betaherpesvirus,
- EHV-3: (Equines Coitalexanthemvirus), den Alphaherpesviren angehörend und
- EHV-4: ebenfalls ein Alphaherpesvirus, vormals als EHV-1 Subtyp 2 bezeichnet.

Die equinen Herpesviren der Serotypen 1 bis 4 verursachen weltweit anhaltend wirtschaftliche Schäden in der Pferdezucht.

Diese entstehen insbesondere durch immer wieder auftretende, seuchenhafte Aborte, Atemwegserkrankungen und zum Teil dramatisch verlaufende Enzephalomyelitiden. Von nicht zu unterschätzender wirtschaftlicher Bedeutung sind die Aufzuchtverluste, Trainingsausfälle und Leistungseinbußen, die vorwiegend durch die respiratorische Verlaufsform der Infektion mit den equinen Herpesviren der Typen 1, 2 und 4 verursacht werden.

Die Eigenschaft aller Herpesviren, nach Primärinfektion im Wirt persistieren zu können, besitzen auch die equinen Herpesviren. Somit spielen latent infizierte Pferde eine wichtige Rolle als Virusreservoir. Durch Stressfaktoren wie Chemotherapie, Geburt, Kastration oder Transport können latente EHV-Infektionen reaktiviert werden. Da eine solche Reaktivierung zwar mit Virusausscheidung verbunden ist, aber klinisch inapparent verlaufen kann, muß jedes einmal infizierte Pferd als potentieller Ausscheider und damit als möglicher Infektionsherd angesehen werden.

Herpesviren zeigen im allgemeinen eine schwach ausgeprägte Immunogenität, die zu einer quantitativ geringen humoralen Immunantwort führt. Speziell die respiratorische Verlaufsform nach EHV-4 Infektion resultiert häufig in einer serologisch schwachen Immunreaktion.

Untersuchungen über die immunogenen Strukturkomponenten von Herpesviren ergaben zunächst, daß nur der Virushülle eine Bedeutung bei der Induktion einer Immunität zukommt. In den letzten Jahren wurden Erkenntnisse über die Bedeutung der einzelnen viralen Glykoproteine von Herpesviren für die Immunreaktion gewonnen. So konnte eine passive Immunität jeweils mit monoklonalen Antikörpern gegen die Glykoproteine gA, gB, gC, gD, gE und gF von Herpes Simplex Viren (HSV) auf Mäuse übertragen werden. Diese Antikörper zeigten zwar keine virusneutralisierenden Eigenschaften in vitro, schützten aber die Mäuse nach Infektion mit einer letalen Dosis von HSV (Balachandran et al., Infection and Immunity 37 (1982) S. 1132 bis 1137).

In Versuchen mit equinen Herpesviren EHV-1 im Hamstermodell wurde Schutz vor homologen Belastungsinfektionen mit lebenden und inaktivierten, kompletten Virionen sowie mit gereinigten kompletten Hüllproteinen erzielt. Dagegen wurde durch die Verabreichung von Fraktionen der Hüllproteine nur ein partieller Schutz durch eine hochmolekulare Fraktion erhalten (Papp-Vid und Derbyshire, Can. J. Comp. Med. 42 (1978) S. 219 bis 226).

Die extrazellulären EHV-Virionen setzen sich aus 28 bis 30 Strukturproteinen zusammen, die, bis auf die 6 Polypeptide des Kapsids, alle in der Virushülle zu finden sind. Die Molekulargewichte dieser Hüllproteine reichen von 16 bis 270 kDa. Die Angaben über die Anzahl der Glykoproteine liegen bei 12 bis 14. Von diesen Glykoproteinen wurden für EHV-1 und EHV-4 sechs als Hauptglykoproteine identifiziert.

Die Molekulargewichte der 6 Hauptglykoproteine, die bei EHV-1 identifiziert wurden, liegen für gp2 bei 252 bis 260 kDa, gp10 bei 127 bis 138 kDa, gp13 bei 92 bis 97 kDa, gp14 bei 81 bis 87 kDa, gp18 bei 60 bis 65 kDa und für gp22 bei 42 bis 46 kDa.

Shimizu et al. Arch. Virol 104 (1989) S. 169 bis 174 beschreiben die Herstellung monoklonaler Antikörper gegen gp13 und gp14 von EHV-1, die, in Hamster appliziert, passive Immunität gegenüber experimentell gesetzter, letaler EHV-1 Infektion vermitteln. Zusätzlich neutralisierten sie verschiedene EHV-1 Stämme in vitro.

Die passive Immunisierung von Hamstern mit Antikörpern läßt keine Rückschlüsse auf die Immunogenität von gereinigten Glycoproteinen im Pferd zu. Die Herstellung der monokionalen Antikörper erfolgte zum einen in Mäusen und ging zum anderen von vollständigen Viren aus.

In der Praxis werden derzeit monovalente EHV-1 Lebendimpfstoffe oder inaktivierte Impfstoffe in Kombination mit anderen Antigenen eingesetzt. Trotz Einsatz dieser Impfstoffe kommt es immer wieder zu klinischen Erkrankungen aufgrund von Infektionen mit EHV-1 und EHV-4. Dies wird zum Teil darauf zurückgeführt, daß der vorgeschriebene, sehr aufwendige Impfplan nicht eingehalten wird, zum anderen sind die Impfstoffe nicht voll befriedigend wirksam, vor allem fehlt Immunschutz gegenüber EHV-4.

Insgesamt ist daher die praktische Situation mit den derzeit eingesetzten Impfstoffen nicht befriedigend.

Es war wünschenswert, zu einer EHV-Vakzine zu kommen, die einfach anzuwenden und gut wirksam ist.

Aus Vaccine Vol. 8 p. 491-496 ist die Herstellung sogenannter ISCOMS mit gereinigten EHV.1 Viruspartikeln bekannt. Über ihre Wirksamkeit im Pferd ist jedoch nichts bekannt.

Aus EP-OS 1 365 ist Herstellung und Verwendung von Proteinen des humanen Herpes Simplex Virus bekannt.

Über ihre Anwendung beim Pferd ist jedoch nichts bekannt.

Stand der Technik im Sinne von Art. 54 (3) ist PCT WO 92/1057, die Glycoproteine gH und gC von EHV-4 und ihre Verwendung als Impfstoffe für Pferde betrifft.

Gegenstand der vorliegenden Erfindung sind:
1. Impfstoffe für Pferde gegen Infektionen mit equinen Herpesviren (EHV), dadurch erhältlich, daß man
   a) equine Herpesviren vermehrt,
   b) die infizierten Zellen oder das extrazelluläre Virus mit Detergentien behandelt,
   c) das so erhaltene Lysat mit Lektinen behandelt,
   d) den Lektin-Ligand-Komplex vom restlichen Lysat abtrennt,
   e) gegebenenfalls den Lektin-Ligand-Komplex aufspaltet und das Glycoproteingemisch isoliert,
   f) anschließend den Lektin-Ligand-Komplex und/oder das isolierte Glycoproteingemisch in üblicher Weise formuliert.
2. Impfstoffe gemäß Anspruch 1, dadurch gekennzeichnet, daß zur Komplexbildung mit den Glycoproteinen Lektine verwendet werden, die Mannose und/oder Glucose sowie deren Konjugate binden.
3. Impfstoffe gemäß Anspruch 1, dadurch gekennzeichnet, daß zur Komplexbildung Lektine aus Canavalia ensiformis oder Helix pomatia sowie deren Kopplungsprodukte an inerte Matrizes eingesetzt werden.
4. Impfstoffe gemäß Anspruch 1, dadurch gekennzeichnet, daß die Glycoproteine aus equinen Herpesviren der Serotypen 1 bis 4 isoliert werden.
5. Komplexe aus Glycoproteinen von equinen Herpesviren oder von Zellen, die mit equinen Herpesviren infiziert wurden und Lektinen sowie deren Koppelungsprodukten an inerte Matrizen.

Die Impfstoffe basieren auf den EH-Virustypen 1 bis 4, insbesondere seien EHV-1 und EHV-4 genannt, bevorzugt ist EHV-1.

Die Impfstoffe lassen sich grundsätzlich aus allen EHV-Virusstämmen herstellen.

Als Beispiele für EHV-Stämme seien genannt
1. Der Stamm RAC-H (EHV-1), ein Virus, das aus einem abortierten Pferdefötus isoliert wurde. Der Stamm wird z.B. auch beschrieben in "Untersuchungen zur Entwicklung eines Lebendimpfstoffes gegen die Rhinopneumonitis (Stutenabort) der Pferde" (Mayr et.al., 1968; Zbl.Vet.Med. Bd.5, 406-408).
2. Der Stamm DA8 (EHV-1) wurde gemäß Budapester Vertrag am 24. 7. 1990 unter der Bezeichnung J-979 beim Institut Pasteur CNCM Paris, Frankreich, hinterlegt.
3. Der Stamm Thein 400/3 (EHV-2) wurde gemäß Budapester Vertrag am 24. 7. 1990 unter der Bezeichung J-981 beim Institut Pasteur CNCM Paris, Frankreich, hinterlegt.
4. Der Stamm Thein 166 (EHV-3)
5. Der Stamm Thein 252/1 (EHV-4) wurde gemäß Budapester Vertrag am 24. 7. 1990 unter der Bezeichnung J-982 beim Institut Pasteur CNCM Paris, Frankreich, hinterlegt.
6. Der Stamm MAR 87 (EHV-1) wurde gemäß Budapest Vertrag am 24.7.1990 unter der Bezeichnung J-980 beim Institut Pasteno CNCM Paris, Frankreich hinterlegt.

Die Glycoproteine zur Herstellung der erfindungsgemäßen Impfstoffe können aus extrazellulärem Virus oder aus infizierten Zellen gewonnen werden. Dazu wird das Virus in an sich bekannter Weise vermehrt.

Die Vermehrung der Viren erfolgt in üblicher Weise in Gewebekulturen animaler Zellen, z.B. in Pferde-Zellen, Schwein-Zellen, Affen-Zellen oder Kaninchen-Zellen, bevorzugt in Pferdehaut-Zellen wie der permanenten Pferde-Haut-Zelle ED (ATCC CCL 57 oder deren Abkömmlingen) oder Schweine-Nieren-Zellen wie der permanenten Schweine-Nieren-Zelle PK15 (ATCC CCL 33 oder deren Abkömmlinge) oder Kaninchen-Nieren-Zellen wie in der Kaninchen-Nieren-Zelle RK-13 (ATCC CCL 37) oder deren Abkömmlingen) oder in Affen-Nieren-Zellen.

Die Vermehrung erfolgt in an sich bekannte Weise in stationären, Roller- oder Carrier-Kulturen in Form von geschlossenen Zellverbänden oder in Suspensionskulturen. Als Vermehrungsmedien für die Zellen werden eingesetzt alle an sich bekannten Zellkulturmedien z.B. beschrieben in Produktkatalog der Fa. Flow Laboratoris GmbH Post 1249, 5309 Meckenheim, wie insbesondere das Minimal Essential Medium (MEM), das als wesentliche Bestandteile Aminosäuren, Vitamine, Salze und Kohlenhydrate enthält, komplettiert mit Puffersubstanzen wie z.B. Natrium-Bicarbonat oder (Hydroxyethylpiperazin-N-2-ethansulfonsäure (Herpes) und gegebenenfalls Tierseren, wie z.B. Seren von Rindern, Pferden bzw. deren Foeten. Besonders bevorzugt wird der Einsatz von foetalem Kälberserum in einer Konzentration von 1-30 Vol-%, vorzugsweise 2-10 Vol.-%.

Die zur Vermehrung der Viren dienenden Zellen und Zellrasen werden in üblicher Weise nahezu bis zur Konfluenz oder bis zu optimaler Zelldichte vermehrt. Vor ihrer Infektion mit Viren wird bevorzugt das Zellvermehrungsmedium entfernt und die Zellen bevorzugt mit Virusvermehrungsmedium gewaschen. Als Virusvermehrungsmedien werden eingesetzt, alle an sich bekannten Zellkulturmedien, wie insbesondere das oben genannte MEM. Danach wird mit einer Virussuspension infiziert. In der Virussuspension liegt das Virus im Virusvermehrungsmedium derart verdünnt vor, daß mit einer MOI (= multiplicity of infection entspricht infektiöse Viruspartikel auf jede vorhandene Zelle) von 0,001-50, bevorzugt 0,10-10 infiziert wird.

Die Vermehrung der Viren erfolgt mit oder ohne Zusatz von Tierseren. Für den Fall, das Serum eingesetzt wird, wird dieses zum Vermehrungsmedium in einer Konzentration von 1-30 Vol.-%, vorzugsweise 2-10 Vol.-% zugegeben.

Infektion und Virus-Vermehrung erfolgt bei Temperaturen zwischen Raumtemperatur und 40° C, bevorzugt zwischen 32 und 39°C, besonders bevorzugt bei 37° C.

Die Vermehrung erfolgt für den Fall, daß mit extrazellufärem Virus weitergearbeitet werden soll bis zur vollständigen Zerstörung des Zellsubstrates. Für den Fall, daß mit Virusproteinen aus infizierten Zellen gearbeitet werden soll, wird die Vermehrung bis zum Erreichen des maximalen Gehalts an virusspezifischen Antigenen durchgeführt. Der maximale Gehalt an virusspezifischen Antigenen ist z.B. bei Monolayerkulturen erreicht bei 30 bis 100 % zytopathogene Veränderungen (zpE). Bevorzugt ist der Bereich um 70 bis 90 % zpE.

Für den Fall, daß mit extrazellulärem Virus gearbeitet werden soll, erfolgt die Virusaufarbeitung durch Enffernen der Zelltrümmer und Isolierung und Konzentrierung der Viruspartikel aus dem Virusvermehrungsmedium. Dies erfolgt in an sich bekannter Weise durch Filtration, wie z.B. Ultrafiltration mit Membran- oder Tiefenfilter, oder durch Zentrifugation. Zum Entfernen von Zelltrümmern wird z.B. eine Zentrifugation zwischen 500 und 15.000 x g, bevorzugt zwischen 5 000 und 15 000 x g durchgeführt. Die Virusisolierung wird erreicht durch Zonenzentrifugation oder isopyknische Zentrifugation in z.B. Saccharose-Dichtegradienten. Hierzu wird z.B. das virushaltige Vermehrungsmedium nach Entfernung der Zelltrümmer einer Zonenzentrifugation bis 100 000 x g bis zur Sedimentation der Viruspartikel unterworfen. Ein höherer Reinheitsgrad wird erreicht, wenn die Zonenzentrifugation zur Pelletierung der Viruspartikel durch eine wäßrige Lösung mit höherer Dichte als das virushaltige Medium durchgeführt wird. Als wäßrige Lösung kann z.B. eine 10-40 % w/w, bevorzugt 25-40 % w/w, gepufferte Lösung von Saccharose dienen.

Eine noch effektivere Reinigung wird durch eine Zentrifugation in einem Dichtegradienten erreicht. Hierzu wird das Virusmaterial, das z.B. durch eine niederförmige Zentrifugation bei 5 000-15 000 x g vorgereinigt und durch eine anschließende Zentrifugation bei 100 000 x g sedimentiert wurde, durch eine zonale oder isopyknische Gradienten-Zentrifugation isoliert. Bevorzugt wird eine isopyknische Zentrifugation durch einen Saccharose-Dichtegradienten von z.B. 30 bis 50 % w/w in gepufferter, wäßriger Lösung bei einer Zentrifugalbeschleunigung von z.B 100.000 bis 150.000 xg. Für den Fall, daß mit Virusproteinen aus infizierten Zellen gearbeitet werden soll, werden die zur Vermehrung der Viren dienenden, infizierten Zellen, falls erforderlich, suspendiert und die suspendierten Zellen konzentriert. Die Konzentrierung erfolgt durch Filtration oder Zentrifugation. Die Zentrifugation erfolgt bevorzugt bei 300 bis 2 000 g. Die Zentrifugation erfolgt bei einem Volumen von ca. 1 l für ca. 15-45 Minuten.

Nach der Konzentrierung werden die Zellen bevorzugt z.B. mit physiologischer Salzlösung gewaschen.

Die so erhaltenen Virus- oder Zellkonzentrate werden mit Detergentien behandelt.

Geeignete Detergentien sind:
anionaktive Tenside, wie Na-Laurylsulfat, Fettalkoholethersulfate, Mono/Dialkylpolyglykoletherorthophosphorsäureester-Monoethanolaminsalz, Calciumalkylarylsulfonat, Natriumdeoxycholat, kationaktive Tenside, wie Cetyltrimethylammoniumchlorid, ampholytische Tenside, wie Di-Na-N-lauryl-:-iminodipropiont oder Lecithin, nicht ionogene Tenside, z.B. polyoxyethyliertes Ricinusöl, polyoxyethyliertes Sorbitan-Monooleat, Sorbitan-Monostearat, Glycerinmonostearat, Polyoxyethylenstearat, Alkylphenolpolyglykolether.

Bevorzugt seien nicht ionogene Detergentien genannt:
nicht-ionische, wasserlösliche Emulgatoren mit einem HLB (hydrophilic/lipophilic/balance-Wert) größer als 10, z.B. Emulgator NP 40® (Bayer AG), Alkylarylpolyglykolether; Renex 678® (Atlas Chemical Industries), Polyoxyethylenalkylarylether; Tween 20® (Atlas), Polyoxyethylensorbitanmonopalmitat; Myri 53® (Atlas), Polyoxyethylenstearat; Atlas G 3707®, Polyoxyethylenlaurylether; Atlas G 3920®, Polyoxyethylene oleylether; Atlas G 9046 T®, Polyoxyethylenmannitanmonolaurat; Emulgator 1371 B® (Bayer AG), Alkylpolyglykolether; Emulgator 1736® (Bayer AG), Alkylpolyglykolether (Oleylpolyglykolether); Emulgator OX® (Bayer AG), Alkylpolyglykolether (Dodecylpolyglykolether); Ninox BM-2® (Stepan Chemical Co.), ethoxyliertes Nonylphenol; Triton X-100® (Rohm an Haas Co.), Isooctylphenolpolyethoxyethanol; Cremophor EL®, Nonidet P 40® (Shell), Glycopyranosid (Sugena), N-decantoyl-N-methyl-glycosid.

Die Detergentien werden in Form verdünnter wässriger Lösungen angewendet. Genannt seien Lösungen mit 0,1 bis 10 Volumenprozent, bevorzugt mit 0,5 bis 5 Volumenprozent, besonders bevorzugt ca. 1 Volumenprozent Detergensgehalt.

Die Detergenslösung wird im Volumenverhältnis ca. 1 : bis ca. 10 : 1 zum Zell- bzw. Viruskonzentrat zugegeben. Bevorzugt ist das Verhältnis Detergenslösung zu Zell- bzw. Viruskonzentrat von ca. 3 : 1.

Zur Verbesserung der Detergensbehandlung kann die Lösung zusätzlich einer Ultraschallbehandlung unterworfen werden.

Die Detergensbehandlung erfolgt bei Temperaturen zwischen 0 und ca. 24°C, bevorzugt zwischen 2 und 8°C. Die Detergensbehandlung dauert 15 Minuten bis 2 Tage, bevorzugt 12 bis 24 Stunden.

Die bei dieser Behandlung nicht gelösten Partikel werden entfernt, bevorzugt durch Zentrifugation oder Filtration. Bei Anwendung der Zentrifugation wird das oben erhaltene Lys at einer Zentrifugal-Beschleunigung von z.B. 100 000 x g für z.B. 1 Stunde ausgesetzt.

Der so erhaltene Zentrifugationsüberstand bzw. das Filtrat kann vor seiner Weiterverarbeitung bei tiefen Temperaturen (0 bis 70°C) gelagert werden.

Zur Isolierung der in der Lösung enthaltenen Glycoproteine wird diese mit Lektinen behandelt. Dazu wird sie zuvor mit soviel Kochsalz sowie den bekannten Lektin-stabilisierenden Salzen versetzt, daß eine Konzentration an Kochsalz von 0,5 bis 2, bevorzugt 0,7 bis 1,2 molar entsteht. Die erforderliche Konzentration der Lektinstabilisierenden Salze ist aus dem Stand der Technik bekannt und für die einzusetzenden Lektine spezifisch. Die Konzentrationseinstellung erfolgt bevorzugt durch Dialyse der Lysate.

Lektine sind Proteine oder Glycoproteine aus Pflanzen, speziell deren Samen, Mikroorganismen, Vertebraten und Invertebraten die spezifisch Zucker und deren Konjugate binden. Erfindungsgemäß verwendet werden Lektine, die Glycoproteine aus EHV-infizierten Zellen bzw. aus EHV-Virionen erkennen und binden und die auf Lymphozyten mitogen wirken.

Grundsätzlich können alle Lektine erfindungsgemäß verwendet werden. Bevorzugt werden Lektine verwendet, die Mannose und/oder Glucose sowie deren Konjugate erkennen.

Im Einzelnen seien genannt die Lektine aus Canavalia ensifornis, Lens culinaris, Lathyros odoratus, Pisum sativum, Vicia faba, Sambucus nigra, Glycine max, Ulex europaens, Helix pomatia, Phytolacca americana, Lycopersicon esculentum, Datura stramonium, Bandeiraea simplicifolia.

Für das erfindungsgemäße Verfahren können die Lektine in wasserlöslicher oder nicht wasserlöslicher Form verwendet werden. In der nicht wasserlöslichen Form werden sie bevorzugt immobilisiert durch Koppelung an inerte Matrizen wie z.B. Dextrane, Agarosen, Cellulosen als Gele eingesetzt.

Im Einzelnen seien genannt Concanavalin-A-Agarose, Concanavalin-A-Sepharose, Lentil-Lectin-Sepharose, Pokeweed-mitogen-Agarose.

Die Lektine werden in Form einer Detergens- und Salzhaltigen Lösung, Suspension oder eines Gels eingesetzt.

Zur Herstellung dieser Lösung, Suspension oder dieses Gels werden die Lektine mit dem zur Behandlung der Zellysate oder Viruslysate eingesetzten Detergens in einer Salzlösung versetzt. Diese Salzlösung enthält ca. 0,5 bis 2, bevorzugt 0,7 bis 1,2 Mol, Kochsalz neben den bekannten und für die Stabilität und Reaktionsfähigkeit der Lektine üblichen und notwendigen Salze. Die Salz- und Detergenslösung der Lektine ist damit identisch mit der Lösung, die zur Einstellung der Salz- und Detergenskonzentration der Zell- und Viruslysate verwendeten Lösung.

Verwendet werden ca. 1 bis 150 mg, bevorzugt 1 bis 50 mg, besonders bevorzugt 5 bis 20 mg, reines Lektin pro ml Lösung, Suspension oder Gel, die oder das Detergens in der bei der Behandlung der Zellysate oder Virionen eingesetzten Konzentration enthält.

Von dieser Lectinlösung, -suspension oder -gel wird den Zellysaten bzw. Viruslysaten, die Detergens enthalten, soviel zugesetzt, daß pro mg Gesamtprotein 0,01 bis 50 mg, bevorzugt 0,1 bis 20 mg, besonders bevorzugt 0,5 bis 5 mg, Lektin eingesetzt werden. In jedem Fall muß die Menge an Lektin so gewählt werden, daß alle Glycoproteine der Zell- bzw. Viruslysate gebunden werden können.

Die Lektinbehandlung erfolgt bei -5 bis +30° C, bevorzugt bei 2 bis 8° C über ca. 10 min bis 3 Tage, bevorzugt bei 1 Stunde bis 2 Tage.

Die Reaktion der Lektine mit den Glycoproteinen kann auch über Säulenchromatographie erfolgen. Dazu werden die immobilisierten Lektine bevorzugt gebunden an eine gelförmige Matrix in einer Chromatographiesäule mit den Zellysaten bzw. Viruslysaten in Kontakt gebracht. Es werden pro ml gepacktes Gel ca. 0,1 bis 100 mg, bevorzugt 1 bis 50 mg, besonders bevorzugt 5 bis 20 mg, Lektin eingesetzt.

Der Glycoprotein-Lektin-Komplex wird in üblicher Weise aus der Gesamtlösung oder -suspension abgetrennt. Dies kann durch Zentrifugation, Filtration oder im Fall der Chromatographie durch Waschen erfolgen.

Die bei diesem Verfahrensschritt erhaltenen Suspensionen bzw. Gele, die die Lektin-Glycoprotein-Komplexe enthalten, werden zur Reduktion der Salz- und Detergenskonzentration gewaschen. Dazu wird die Suspension oder das Gel mit einer Lösung, die das 0,1 bis 0,01-fache der Detergenskonzentration und Kochsalzkonzentration enthält, die bei der Behandlung des Zell- oder Viruslysates eingesetzt wurde, versetzt, so daß die Konzentration der Lektin-Glycoprotein-Komplexe enthaltenden Suspension oder Gels an Detergens und Kochsalz auf das 0,1 bis 0,01-fache reduziert ist.

Die so erhaltenen Suspensionen oder Gele der Lektin-Glycoprotein-Komplexe können direkt oder gegebenenfalls nach Adjuvierung zur Immunisierung von Pferden eingesetzt werden. In Abhängigkeit vom Gehalt des an Lektin gebundenen Glycoproteins können die zur Immunisierung eingesetzten Suspensionen oder Gele weiter konzentriert oder verdünnt werden. Der Gehalt an gebundenem Glycoprotein wird so eingestellt, daß pro Impfdosis pro Pferd 10 bis 250 mg, bevorzugt 10 bis 100 mg, besonders bevorzugt 25 bis 50 mg, Glycoprotein enthalten sind.

In den erhaltenen Suspensionen oder Gelen der Lektin-Glycoprotein-Komplexe kann durch Filtration, Zentrifugation, Dialyse oder andere Waschvorgänge die Konzentration des Detergens bis hin zu seiner Eliminierung verändert werden. Mit den gleichen Methoden kann auch die Salzkonzentration im physiologisch verträglichen Bereich verändert werden.

Die Suspensionen oder Gele der Lektin-Glycoprotein-Komplexe können bei Temperaturen unter 8° C gelagert werden. Sie lassen sich auch gefriertrocknen.

Aus den erhaltenen Suspensionen oder Gelen der Glycoprotein-Lektin-Komplexe können zur Herstellung des Impfstoffes die Glycoproteine isoliert werden. Dazu werden die Suspensionen oder Gele mit einer zuckerhaltigen, wässrigen Salzlösung behandelt.

Die Art des einzusetzenden Zuckers richtet sich nach der Spezifität der verwendeten Lektine. Die Konzentration des Zuckers beträgt 0,1 bis 1 molar, bevorzugt 0,1 bis 0,5 molar, besonders bevorzugt 0,3 bis 0,5 molar. Konzentration und Zusammensetzung des Salzgehaltes und Detergensgehaltes der Zuckerlösung entspricht der der Glycoprotein-Lektin-Komplexe enthaltenden Gele oder Suspensionen.

Die Behandlung mit der Zuckerlösung erfolgt bei -5 bis +30° C, bevorzugt zwischen 2 und +8° C. Die Behandlung beträgt ca. 15 min bis 4 Tage, bevorzugt 1 Stunde bis 2 Tage, besonders bevorzugt 10 bis 24 Stunden.

Die hierbei eluierten Glycoproteine werden durch Zentrifugation, Filtration oder durch andere übliche Trennverfahren (z.B. Chromatographie) von den Lektinen isoliert.

Die so erhaltenen isolierten Glycoproteine in Detergens-Salz- undr zuckerhaltiger Lösung können in Abhängigkeit des Glycoproteingehalts direkt oder nach Adjuvierung zur Immunisierung von Pferden eingesetzt werden.

In Abhängigkeit vom Gehalt an Glycoproteinen können die zur Immunisierung eingesetzten Lösungen konzentriert oder verdünnt werden. Der Gehalt an Glycoproteinen wird so eingestellt, daß pro Impfdosis pro Pferd 10 bis 250 mg, bevorzugt 10 bis 100 mg, besonders bevorzugt 25 bis 50 mg, Glycoprotein enthalten sind.

Bei Präparationen aus Viruslysaten kann der Gehalt an Glycoprotein so eingestellt werden, daß er pro Impfdosis um das 0,1 bis 0,01-fache niedriger liegt.

In den Präparationen lassen sich die Konzentrationen von Detergens und Salzen wie weiter oben beschrieben verändern. Die Lagerung der Präparationen erfolgt in Form ihrer Lösungen bei tiefen Temperaturen (unter 0° C) oder in lyophilisierter Form.

Die Lektin-Glycoprotein-Komplexe und die isolierten Glycoproteine können zur Steigerung der Immunantwort vor ihrer Verabreichung mit üblichen Adjuvantien versetzt werden.

Als Adjuvantien seien genannt: wässrige und ölige Adjuvantien wie z.B. Aluminiumhydroxide oder ® Havlogen.

Die Verabreichung erfolgt in üblicher Weise parenteral wie intramuskulär, subkutan, intranasal.

### Beispiele

### 1. Herstellung der Zellkulturen

### 1.1 Medien und Lösungen

- Anzuchtmedium:: E-MEM 0,85 g (Earle Minimal Essential Medium) (vergl. Virol. Arbeitsmethoden, Bd.1, Gustav Fischer-Verlag, Stuttgart 1974, A. Mayer, P.A. Bachmann, B. Bibrack + G. Wittmann) + 0,85 g NaHCO₃/l
- Trypsin EDTA:: 0,2 g EDTA-Ethylendiaminotetraessigsäure-natriumsalz; 1 g Glucose; 0,4 g KCI; 8 g NaCI; 0,58 g NaHCO₃; 0,5 g Trypsin auf 1 l Aqua dest.
Foetales Kälberserum (FCS): Fa. "Gibco"

### 1.2 Zell-Anzucht

Die Virusanzüchtungen sowie die Virus-Titrationen wurden mit ED (equine dermal) und PK15-Zellen (porcine kidney) durchgeführt. Als Medium zur Anzucht der Zellen fand E-MEM 0,85 g unter Zusatz von 10 % foetalem Kälberserum Verwendung. Die Zellen wurden bei Konfluenz durch Trypsin-EDTA aus dem Monolayerverband gelöst und in einer dem Anzuchtgefäß entsprechenden Dichte passagiert.

### 2. Herstellung der Viruskulturen

### 2.1 Medien und Lösungen

- Erhaltungsmedium:: E-MEM (2,0 g (Earle Minimal Essential Medium) (Virol. Arbeitsmethoden, Bd.1, Gustav Fischer-Verlag, Stuttgart 1974, A. Mayer, P.A. Bachmann) + 2,0 g NaHCO₃/l)
- PBS:: 8 g NaCI, 0,2 g KH₂PO₄, 1,44 g Na₂HPO₄, 0,2 KCI auf 1 l Aqua dest.

### 2.2 Virus-Anzucht

Das Anzuchtmedium der Zellen wurde bei Konfluenz dekantiert und die Zellen mit Erhaltungsmedium gewaschen. Die anschließende Infektion erfolgte durch Zugabe des in Erhaltungsmedium verdünnten Virus-Stocks. Dabei wurde EHV-1 1:100, entsprechend einer m.o.i. (multiplicity of infection) von 0,1 und EHV-4 1:50 (in einer m.o.i. von 0,001) verdünnt. Inkubiert wurden die inokulierten Zellkulturen bei 37° C. Die mit EHV-1 beimpften Zellen erreichten einen 100 %igen zpE (zytopathischen Effekt) nach etwa 24 Stunden post infectionem, während die EHV-4-infizierten Zellen mindestens 48 Stunden benötigten.

### 3. Herstellung der Zellysate

### 3.1 Chemikalien und Lösungen

- Extraktionspuffer:: 25mM TrisHCI(Tris(hydroxymethyl)-aminomethan-HCI), 1 % NP40; pH 8,6
- NP40:: Nonident P 40 (Ethylphenylpolyethylenglycol)
- Lagerungspuffer:: 0,1M NaCH₃COO, 1mM CaCl₂, 1mM MgCl₂, 1mM MnCl₂, 1M NaCl, pH 6
- Elutionspuffer:: 0,1M NaCH₃COO, 1mM CaCl₂, 1mM MgCl₂, 1mM MnCl₂, 0,1M NaCI, 0,1% NP40, 0,3M Methylmannopyranosid, pH 6

### 3.2 Detergensbehandlung

Die in Rollflaschen (850 cm² Falcon) oder Wannenstapel (6000 cm² Nunc) angezogenen ED-Monolayerzellkulturen wurden bei einer Konfluenz von 90 bis 100 % mit EHV-1 beziehungsweise EHV-4 beimpft. Nach den Ergebnissen von Kinetikuntersuchungen (Ernten bei einem zpE von 10 bis 100 %) wurden für die präparativen Experimente die infizierten Zellen bei einem zpE von 80 % geerntet. Dafür wurden die Zellen aus Rollerkulturen mittels eines sterilen Gummischabers (cell-scraper, Fa. Costar) von den Gefäßwänden in das Medium abgeschabt beziehungsweise durch starkes Schütteln der Wannenstapel in das Kulturmedium suspendiert. Anschließend wurde die Suspension bei 3.000 g für 20 min zentrifugiert und das so gewonnene Zellpellet 2 x in PBS gewaschen, bevor es schließlich 1:2 in PBS v/v aufgenommen und bei -70° C gelagert wurde. Die Extraktion erfolgte durch Zugabe von 3 ml Extraktionspuffer pro 1 ml Zellsuspension und anschließender Ultraschallbehandlung (3 Stöße von je 5 sec. bei 7 microns). Dieses Zellysat wurde für 24 Stunden bei 4° C gerührt, bevor durch Zentrifugation im SW41-Rotor (38.000 rpm, 1 Stunde) unlösliche Partikel durch Sedimentation entfernt wurden. Das Überstandsmaterial wurde bei -70° C gelagert. Die Bestimmung der Proteinkonzentration in den Membranlysaten erfolgte nach Ultrafiltration eines Aliquots des Überstandes.

### 4. Isolierung von Glykoproteinen

Die Reinigung von Glykoproteinen an Con A-Sepharose mittels Affinitätschromatographie wurde von TODD et al., Arch. Virol 96 (1987), S. 215-224, zur Präparation von Glykoproteinen des Aujeszky Virus beschrieben. Die Zell-Lysate wurden zunächst bei 4°C für 24 Stunden gegen den Lagerungspuffer der Con A-Sepharose 4B, komplettiert mit dem Detergens NP40 (1 % Endkonzentration), dialysiert. Die Concanavalin A-Sepharose 4B (Sigma C9017) wurde blasenfrei in eine Säule (Pharmacia 1,5 cm Durchmesser) gebracht und mit dem 20fachen Bettvolumen des oben beschriebenen Lagerungspuffers (+1 % NP40) bei 4° C equilibriert. Anschließend erfolgte die Adsorption der Glykoproteine an das Lektin durch die Applikation der Zell-Lysate auf die Säule (15 mg Protein/ml gepacktes Gel). Dazu wurde das Zell-Lysat (durchschnittlicher Proteingehalt 2,1 mg/ml) mittels Peristaltikpumpe mit einer Pumpgeschwindigkeit von 1 ml/Stunde in das Gel eingebracht und für 12 Stunden stationär inkubiert. Die Säule wurde im Anschluß hieran mit Equilibrierungspuffer gespült, um nicht gebundenes Protein abzuwaschen. Diese, sowie alle folgenden chromatographischen Arbeiten wurden bei ca. 4° C durchgeführt. Zur Reduktion der Detergenz- und Salzkonzentration wurde das mit Glykoproteinen adsorbierte Gel mit dem 20fachen Säulenvolumen eines modifizierten Equilibrierungspuffers mit reduziertem NaCI- (0,1 M) und NP40-Gehalt (0,1 %) erneut equilibriert. Die Elution der Glykoproteine erfolgte mit diesem reduzierten Puffer nach Zugabe von 0,1 bis 0,5 M Methylmannopyranosid. Der Elutionspuffer wurde mit einer Pumpgeschwindigkeit von 2 bis 5 ml/Stunde in das Gel eingebracht und die Säule anschließend über Nacht mit dem Elutionspuffer stationär inkubiert. Anschließend wurde das Eluat unter photometrischer Kontrolle (275 nm) im Durchflußphotometer mit einer Pumpgeschwindigkeit von 2 ml/Stunde gewonnen. Da selbst mit hohen Methylmannopyranosidkonzentrationen die adsorbierten Glykoproteine nicht in einem Elutionslauf von dem Lektin quantitativ gelöst werden konnten, wurde eine weitere Elution durch nochmalige stationäre Inkubation des Gelbettes im Elutionspuffer durchgeführt. Dieser zweite Elutionslauf erbrachte in Qualität und Quantität vergleichbare Glykoproteinmengen. Gelagert wurden diese Glykoprotein-Eluat-Fraktionen bei -20° C. Routinemäßig wurde aufgrund der Ergebnisse in den Optimierungsversuchen der oben beschriebene Elutionspuffer mit 0,1M NaCl, 0,1 % NP40 und 0,3m Methylmannopyranosid eingesetzt.

### 5. Proteinbestimmung

Da das im Zell-Lysat mit 1 % und in den Eluaten mit 0,1 % enthaltene NP40 die photometrische Proteinbestimmung erschwerte, mußte das NP40 durch eine vorausgehende Ultrafiltration in der Probe verdünnt werden. Hierfür wurde ein Aliquot von 50 µl (bei Lysaten mit 1 % NP40) beziehungsweise 500 µl (bei Eluaten mit 0,1 % NP40) der zu bestimmenden Probe in Centricon 10 Tubes (Fa. Amicon) auf 2 ml mit destilliertem Wasser aufgefüllt und bei 8.000 rpm (Rotor A8.24) für 30 min zentrifugiert. Nach zweimaligem Waschen des Retentats mit je 2 ml Wasser wurde dieses auf exakt 1 ml mit destilliertem Wasser aufgefüllt und der Proteingehalt nach Zugabe von Bio-Rad-Protein-assay bei 595 nm photometrisch bestimmt. Hieraus ließ sich durch einfache Rechnung der Proteingehalt der Ausgangsprobe berechnen.

### 6. Präparation von an Lektin gebundenen Glykoproteinen

Gepacktes Concanavalin-A-Sepharose 48 (Sigma C9017) wurde fünfmal mit dem jeweils zweifachen Volumen Lagerungspuffer (komplettiert mit 1 % NP40) in Falcon-tubes durch Pelletieren (3.000 rpm) und Resuspendieren gewaschen, bevor das gegen den gleichen Puffer dialysierte Zell-Lysat zugegeben wurde. Die Adsorption der Glykoproteine an das Lektin erfolgte unter leichtem Schwenken auf einem Taumler für 16 Stunden bei 4° C. Nicht gebundenes Protein wurde anschließend durch wiederholtes Waschen im zweifachen Volumen Lagerungspuffer (+1 % NP40) entfernt. Zur Reduktion des Salzgehaltes und zur Eliminierung des Detergenz wurde das Lektin zunächst mit dem modifizierten Lagerungspuffer (mit 0,1M NaCI und 0,1 % NP40) und anschließend im gleichen Puffer ohne NP40 wiederholt gewaschen. Diese Suspension des mit Glykoproteinen adsorbierten Lektins wurde bei 4° C gelagert. Die Quantifizierung der an Con-A gebundenen Proteine wurde wie folgt durchgeführt: Ein Aliquot von 200 µl des mit Glykoproteinen gebundenen Lektins wurde zweimal mit je 500 µl Elutionspuffer (Lagerungspuffer mit 0,1 M NaCI, 0,5M Methylmannopyranosid) für jeweils 16 Stunden inkubiert. Die Proteinmenge der dabei gesammelten Eluate wurde photometrisch bestimmt und durch einfache Rechnung auf die an 1 ml gepacktem Con-A gebundene Proteinmenge hochgerechnet.

### 7. Analysen zur Antigenität

- Transferpuffer:: 50 mM Tris(hydroxymethyl)aminomethan
40 mM Glycin
1,3 mM Na-Dodecylsulfat
20 % Methanol
pH 9,3
- Probenpuffer:: 15 mM Tris(hydroxymethyl)aminomethan
2 % w/v Na-Dodecylsulfat
10 % w/v β-Mercaptoethanol
6 M Harnstoff
10 % Glycerin
0,02 % w/v Bromphenolblau
pH 6,8

### 7.1 Elektrophoretische Auftrennung der Proteine

Die SDS-Page (Sodium Dodecyl Sulfat-Polyacrylamidgel-elektrophorese) wurde nach der von Lämmli, Nature 227 (1970), S. 680 - 685, beschriebenen Methode durchgeführt. Um eine ausreichende Trennung aller in den Lösungen befindlichen Proteine zu erreichen, wurden 7,5 % bis 15 % Polyacrylamid-Gradientengele verwendet.

Die zu untersuchenden Proben wurden unter Vakuum eingeengt und in jeweils 25 µl eines denaturierenden Probenpuffers resolubilisiert. Nach einer Ultraschallbehandlung von 5 min wurden sie für 4 min aufgekocht und in die Probentaschen aufgetragen.

Als Markerproteine dienten Bio-Rad "Prestained SDS-Page low range standards" mit Proteingewichten von 110, 84, 47, 33, 24 und 16 kDa.

Nach der Elektrophorese wurden die SDS-Polyacrylamid-Gele für 16 Stunden in einer Coomassie-Färbelösung unter leichtem Schütteln bei Raumtemperatur gefärbt. Nicht-proteinspezifische Färbungen wurden anschließend eleminiert durch 2 bis 3stündige Inkubation in einer Entfärbelösung. Die Gele wurden dann unter Vakuum und Hitze auf Filterpapier getrocknet.

### 7.2 Transfer der aufgetrennten Proteine auf Nitrozellulose

Nach Auswertung des gefärbten Gels wurden in der SDS-Page aufgetrennte Proteine auf Nitrozellulosemembrane überführt nach dem von Burnette A., Anal. Biochem 112 (1981), S. 195 -203, beschriebenen Prinzip).

Das Gel wurde zunächst in dem Transferpuffer equilibriert. Dann wurde in der Reihenfolge Filterpapier, Gel, Nitrozellulose, Filterpapier ein Sandwich hergestellt und blasenfrei auf die Kathode des Sartoblots II S (Sartorius) gelegt und nach Auflegen der Anodenplatte der Transfer gestartet. Nach 1 Stunde bei Zimmertemperatur und konstant 1 mA/cm² Gel wurde gestoppt.

### 7.3 Immunfärbung

Die zu färbenden Nitrozellulosestreifen wurden zunächst zur Abdeckung freier Proteinbindungsstellen 1 Stunde in dem Blocking-Puffer (3 % Bovines Serumalbumin (BSA), 0,05 % Tween 20 in PBS) geschwenkt. Anschließend wurde die Nitrozellulose mit den Antiseren in ihren spezifischen Verdünnungen (1:150 bis 1:10.000) im gleichen Puffer für 2 Stunden bei Raumtemperatur inkubiert. Nach gründlichem Waschen mit PBS wurde das, den verwendeten Antiseren entsprechende, mit Peroxidase markierte Anti-Spezies-IgG Konjugat (Firma Sigma), in einer Verdünnung von 1:150 in PBS auf die Nitrozellulose gegeben und diese für weitere 2 Stunden inkubiert. Nach dreimaligem Waschen in PBS wurde die Substratlösung zugesetzt. Diese wurde frisch vor Gebrauch mit 10 mg 3-Amino-9-Ethylcarbazol, 3 ml Dimethylsulfoxid (DMSO) in 50 ml 20 mM Na-Acetat angesetzt. Die Farbreaktion wurde durch Wässern in dest. Wasser abgebrochen. Nach Bestimmung der Laufstrecke der Markerproteine im Trenngel wurden diese Werte gegen ihre zugehörigen Molekulargewichte halblogarithmisch aufgetragen und mittels dieser, für jedes Gel spezifischen, Eichkurve die Molekulargewichte der immungefärbten Proteine ermittelt.

Als Pferdeseren für die immunologischen Untersuchungen fanden Seren folgender Tiere Verwendung:
- "Maienschein": ein mit experimentell hergestellter, inaktivierter, bivalenter Vakzine (EHV-1 und EHV-4) immunisiertes Warmblutpferd, mit hohem EHV-1 SN-Titer (1:112) und niedrigem EHV-4 SN-Titer (1:6).
- "Arfe": ein Pferd des später (8.) beschriebenen Versuches mit hohem EHV-1 SN-Titer (1:80) und geringem, kreuzreagierendem EHV-4 SN-Titer (1:6).
- "Ellis": eine in den USA mit EHV-1 experimentell infizierte Stute, die im Verlauf dieser Infektion abortierte, mit hohem EHV-1 SN-Titer (1:144) und geringem EHV-4 SN-Titer (<1:8).
- "Feldserum": ein mit respiratorischen Symptomen klinisch erkranktes Reitpferd, mit mittelgradig hohem EHV-1 SN-Titer (1:64) und EHV-4 SN-Titer (1:14).

Monospezifische Ziegenseren wurden aus Tieren gewonnen, die gegen verschiedene EHV-1 Stämme oder EHV-4 hyperimmunisiert waren.

Mit Kaninchen der Rasse "große weiße Neuseeländer" wurden monospezifische Hyperimmunseren gegen EHV-1 mit bandengereinigtem Virusmaterial hergestellt. Hierzu wurden 3 x in 14-tägigem Abstand je 100 µg gelöstes Virusprotein/Dosis homogen mit Freund'schem inkompletten Adjuvans formuliert und dem Kaninchen subkutan appliziert. Ebenfalls in 2-wöchigen Intervallen wurde Serum gewonnen.

### 8. Immunogenitätsstudien am Pferd

### 8.1 Vakzineherstellung

### 8.1.1 Herstellung der Eluatvakzine

Nach photometrischer Bestimmung des Proteingehalts der Glykoprotein-Eluat-Fraktionen wurden diese mit sterilfiltriertem Elutionspuffer auf den gewünschten Proteingehalt pro Impfdosis von 2 ml eingestellt. Durch die Zugabe von 0,2 ml R Halogen D als Adjuvans (entspricht 10 %) sowie 0.001 % Merthiolat zur Konservierung, wurden die Vakzineformulierungen komplettiert und anschließend auf pH-Wert und Sterilität geprüft. In dieser Weise wurden folgende Versuchsvakzinen hergestellt:
- EHV-1:: A1: 12,5 µg Protein / Dosis (=2ml)
A2: 75,0 µg Protein / Dosis (=2ml)
A3: 225,0 µg Protein / Dosis (=2ml)

### 8.1.2 Herstellung der Lektinvakzine

Die Suspension des mit Glykoproteinen komplexierten Lektins wurde durch Zugabe von sterilfiltriertem PBS auf die gewünschte Proteinkonzentration der Glykoproteine pro Applikationsdosis verdünnt. 10 % Havlogen D als Adjuvans und 0,001 % Merthiolat zur Konservierung komplettierten die Formulierungen.

Folgende Versuchsvakzinen wurden in Analogie zu den Konzentrationen der Eluatvakzine präpariert:
- EHV-1:: B4: 12,5 µg Con-A gebundenes Protein / Dosis (= 2 ml)
B5: 75,0 µg Con-A gebundenes Protein / Dosis (= 2 ml)
B6: 225,0 µg Con-A gebundenes Protein / Dosis (= 2 ml)

### 8.2 Versuchstiere

Für die Untersuchungen standen uns 26 Jährlinge zur Verfügung. Die Herde bestand aus Warmblutpferden der

Rasse "Deutsches Reitpferd" und Vollblutarabern.

### 8.3 Immunisierungsschema

Die für die Versuche vorgesehenen Pferde wurden zunächst im Serumneutralisationstest auf ihren Immunstatus gegenüber EHV-1 und EHV-4 geprüft. Danach wurden homogene Gruppen zusammengestellt, und zum Datum der Primovakzination die Serumproben der O-Proben gewonnen. In der EHV-1-Studie erfolgte die Grundimmunisierung der 6 Gruppen mit jeweils 4 Pferden durch zweimalige Impfung im Abstand von 5 Wochen. Dabei erhielten die Tiere der Gruppe A1 zur Primovakzination und Boosterung je 12,5 µg, die der Gruppe A2 75 µg und die der Gruppe A3 225 µg der eluierten und mit Havlogen adjuvierten Glykoproteine. Entsprechend wurden die Pferde der Gruppen B4 bis B6 mit der Lektinvakzine immunisiert. Nach 27 Wochen erfolgte die, für alle Gruppen einheitliche, Revakzination mit einer Eluatformulierung (75 µg Protein / Dosis).

Die Injektion der Vakzine erfolgte tief intramuskulär in den M. pectoralis. 2 Tiere wurden als ungeimpfte Kontrollen geführt. Während des gesamten Verlaufs des Versuchs wurden in 5 bis 7-wöchigen Abständen weitere Serumproben gezogen.

### 8.4 Untersuchung auf klinische Verträglichkeit

Zunächst wurde in einem Vorversuch die Verträglichkeit beider Formulierungen geprüft. Elutionspuffer (2 ml/Dosis) einerseits sowie gepackte Con-A Sepharose (5 µl/Dosis) andererseits, jeweils ohne virale Proteine und Adjuvans, wurde je 3 Tieren appliziert. In stündlichen Intervallen wurden Körpertemperatur, Herz, Kreislauf, Hauttrugor, Kopf, Schleimhäute, Allgemeinsymptone sowie auf eventuelle lokale Reaktionen an der Injektionsstelle untersucht. Hierbei wurden in keinem dieser Parameter von der physiologischen Norm abweichende Befunde erhoben. Auch die im Verlauf der Immunogenitätsstudien durchgeführten Verträglichkeitskontrollen zeigten mit Ausnahme eines Tieres der Gruppe B5 (mit geringradiger lokaler Reaktion 24 Stunden post vaccinationem) keine Impfreaktion lokaler oder systemischer Art.

### 8.5 Serologische Untersuchungen im SNT

Die Seren wurden auf neutralisierende Antikörper (Serumneutralisationstest SNT) gegen EHV-1 und EHV-4 untersucht. Dies geschah nach der Serumverdünnungsmethode in Mikrotiterplatten. Die zu untersuchenden Serumproben wurden nach Inaktivierung (30 Minuten bei 56° C) mit einer Reaktionsmenge von 25 µl in Zweierpotenzen mit E-MEM 2,0 g verdünnt und pro Verdünnungsstufe in 4 Näpfe vorgelegt. Nach Zugabe einer Virusdosis von 100 KID₅₀ in 25 µl pro Napf wurde 2 Stunden bei 37° C inkubiert. 50 µl der Zellsuspension mit 300 x 10³ Zellen pro ml (PK15-Zellen im EHV-1 SNT und ED-Zellen im EHV-4 SNT) wurden pro Napf zugegeben und die Platten 7 Tage unter 5 % CO₂-Begasung und 37° C bebrütet. Die Ablesung des zpE erfolgte am 4. und 7. Tag. Ausgewertet wurde nach Kärber, Naunyn-Schmiedebergs Arch. exp. Path. Pharm 162 (1931) S. 480, beziehungsweise nach Reed und Muench, Am. J. Hyg. 27 (1938) S. 493.

### Ergebnisse

### 1. Kinetik der Expression von Membranantigenen EHV-1 und EHV-4 infizierter Zellen

Um den optimalen Erntezeitpunkt für die Präparation von virusspezifischen Antigenen aus infizierten Zellkulturen zu bestimmen, wurde der zeitliche Verlauf der Inkorporation virusspezifischer Proteine in die Zellmembranen kinetisch untersucht. Hierfür wurden infizierte Zellen zu verschiedenen Zeitpunkten post infectionem geerntet und mit NP40 lysiert. Im Anschluß hieran wurden die detergenzlöslichen Proteine dieser Lysate parallel zu den von Zell-Lysaten uninfizierter Zellen in der reduzierenden SDS-Page aufgetrennt. Nach Elektrotransfer dieser Proteine auf Nitrozellulosestreifen folgte eine Immunfärbung, um ihre Antigenität zu analysieren.

Durch die Immunfärbung der Lysatproteine EHV-1-infizierter Zellen bei einem Erntezeitpunkt von 35 %, 75 % und 90 % zpE mit dem Serum "Maienschein", ließ sich die Abhängigkeit des Antigengehaltes vom Erntezeitpunkt verifizieren. Während im Immunoblot der Lysatpräparation mit dem Erntezeitpunkt von 35 % zpE nur zwei Antigene mit 82 und 44 kDa schwach detektierbar waren, ließen sich in dem Lysat, das bei 75 % zpE gewonnen wurde, sieben virusspezifische, antigene Proteine nachweisen. Die relativen Molekulargewichte dieser Proteine lagen bei 137, 96, 86 bis 78, 56, 44, 35 und 27 kDa. Dominant färbten sich die Banden mit 86 bis 78 und 44 kDa. Die gleichen Antigene, wenn auch in reduzierter Menge, konnten auch in dem Membranlysat mit dem Erntezeitpunkt 90 % zpE dargestellt werden. Zusätzlich zeigten sich in allen Präparationen noch Proteinbanden mit 60 und 48 kDa, die auch in der Negativkontrolle nachweisbar waren.

Das Ergebnis dieser Kinetik ergab, daß die Ausbeute an viralen Antigenen sowohl qualitativ als auch quantitativ in den Lysaten EHV-1-infizierter Zellen bei einem Erntezeitpunkt 75 % zpE am höchsten war. Zu einem späteren Zeilpunkt der Infektion (90 % zpE) reduzierte sich der Antigengehalt in den Präparationen wieder. Aus diesen reproduzierten Kinetikuntersuchungen wurde so ein Erntezeitpunkt der infizierten Zellen von etwa 80 % zpE ermittelt, um eine optimale Ausbeute an EHV-1 Membranantigenen zu erhalten.

Das Lysat von EHV-4-infizierten Zellen, die bei einem zpE von 10 % geerntet wurden, enthielt noch keine von dem monospezifischen EHV-4-Ziegenserum nachweisbaren virusspezifischen Antigene. In Lysaten, die bei 40 % zpE präpariert wurden, konnten dagegen zwei virusspezifische Proteine mit Molekulargewichten von 62 und 44 kDa als Antigene nachgewiesen werden. Zusätzlich zu diesen dominanten Antigenen bandeten in Lysaten mit fortschreitendem zpE von 60 % und 90 % noch weitere EHV-Antigene mit Molekulargewichten von 58, 56, 37 und 25 kDa sowie nur schwach erkennbar bei 140 kDa. Aufgrund dieser Untersuchung wurde auch für die routinemäßige Gewinnung von EHV-4 Membranantigen ein Erntezeitpunkt von ca. 80 % zpE gewählt.

Ein Großteil der Virusantigene, die bereits in extrazellulären Virionen von EHV-1 identifiziert worden waren, konnte auch in den Lysaten infizierter Zellen nachgewiesen werden. Dabei waren die Antigene mit Molekulargewichten von 140, 98 bis 88, 84 bis 78, 44 und 27 kDa in beiden Antigenpräparationen vorhanden.

Dagegen differierte das Antigenmuster bei EHV-4 in Abhängigkeit von der Antigenpräparation. Die aus infizierten Zellen gewonnenen Membranproteine repräsentierten virusspezifische Antigene mit Molekulargewichten von 27 bis 62 kDa. Somit fehlten in diesen Membranproteinpräparationen die höher molekularen Antigene (140 bis 70 kDa), die mit dem monospezifischen EHV-4 Serum in extrazellulärem Virus erkannt wurden.

### 2. Isolierung von Glykoproteinen aus Membranlysaten EHV-1 und EHV-4 infizierter Zellen

In einem vorgeschalteten Screening wurden Concanavalin-A (Con-A), Helix pomatia und Phytolacca americana zur Auswahl des für diese Untersuchungen geeigneten Lektins in der Affinitätschromatographie überprüft. H. pomatia und P. americana adsorbierten nur einen geringen Anteil der als antigen erkannten Membranproteine. Dagegen erbrachte Con-A sowohl qualitativ als auch quantitativ die höchste Ausbeute an virusspezifischen Antigenen. Dementsprechend wurde im Weiteren Con-A in der Lektin-Affinitätschromatographie verwendet.

Um die Effektivität des Reinigungsprozesses zu verifizieren und die Antigenität der gereinigten Glykoproteinfraktion zu analysieren, wurden die Proteine der Membranlysate, des Durchflusses der Chromatographie (nicht an das Lektin adsorbierte Proteine) und der Eluate EHV-1-infizierter Zellen im Immunoblot getestet.

Trotz gleicher Proteinmengen der Proben färbten sich mit dem Serum "Maienschein" in den Eluaten die jeweiligen Antigene stärker als in den entsprechenden Lysaten. Dies bedeutet, daß der relative Gehalt EHV-1 spezifischer Antigene in den Eluatpräparationen höher war als in den Lysaten. Dabei reagierten besonders 4 Proteine mit Molekulargewichten von 96 bis 88, 78 bis 76, 62 und 46 bis 42 kDa in diesem Immunoblot. Zusätzlich wurden in den Eluatproben schwach nachweisbare Antigene bei 135 und 27 kDa erkannt, die in den Lysaten nicht nachgewiesen werden konnten, also ebenso im Eluat angereichert wurden. Weiterhin zeigte sich, daß in dem Durchfluß der Chromatographie als einziges Antigen ein mit den Antikörpern des Serums "Maienschein" reagierendes Protein von 62 kDa enthalten war. Dieses Protein ließ sich allerdings auch in dem Lysat, dem Durchfluß und dem Eluat uninfizierter Zellen nachweisen, was dafür spricht, daß es sich hierbei um ein Zellmembranprotein handelte.

Bei dem Vergleich der Membranantigene in den Lysaten und Eluaten EHV-4 infizierter Zellen im Immunoblot wurden in beiden Präparationen die gleichen Proteine von dem eingesetzten anti-EHV-4-Ziegenserum als Antigen erkannt. Es handelte sich hier um Proteine mit Molekulargewichten von 62, 56, 54, 44 und 27 kDa, wobei das Antigen bei 62 kDa quantitativ dominierte. Zusätzlich waren in der Eluatpräparation sehr schwach dargestellte Banden bei 76 und 35 kDa detektierbar.

Wie diese Versuche zeigen, konnte durch die Lektin-Affinitätschromatographie mit Concanavalin-A eine Reinigung von Membranglykoproteinen aus Lysaten EHV-infizierter Zellen erreicht werden, ohne deren antigene Strukturen zu verändern. Die Antigenmuster der Eluate glichen im wesentlichen denen der Lysate. Außerdem belegte die im Vergleich zu den Lysaten deutlich verstärkte Farbreaktion der antigenen Proteine in den Eluaten bei Einsatz in der Immunfärbung einen vermehrten Gehalt und somit eine Anreicherung dieser Antigene in den Eluatpräparationen.

Die Proteinmuster, ohne Berücksichtigung der Antigenität der einzelnen Präparationen vor und nach der Reinigung, wurden durch die proteinspezifische Coomassiefärbung der elektrophoretisch aufgetrennten Proteine analysiert. Während in den Membranlysaten noch mindestens 30 Proteine vorlagen, reduzierte sich die Anzahl der mit Coomassie nachweisbaren Proteine in den Eluaten auf ca. 12. Dieses Ergebnis gilt für die jeweiligen Präparationen von EHV-1 als auch von EHV-4 infizierten Zellen gleichermaßen.

Es konnte damit gezeigt werden, daß durch diese dargestellte Methode bei einer Reduzierung der Gesamtzahl verschiedener Proteine, eine Erhöhung des Antigengehaltes in den Eluaten erreicht werden konnte. Tabelle 1 gibt einen schematischen Überblick zur Bilanz des Gehaltes einzelner Glykoproteine während der Reinigung.

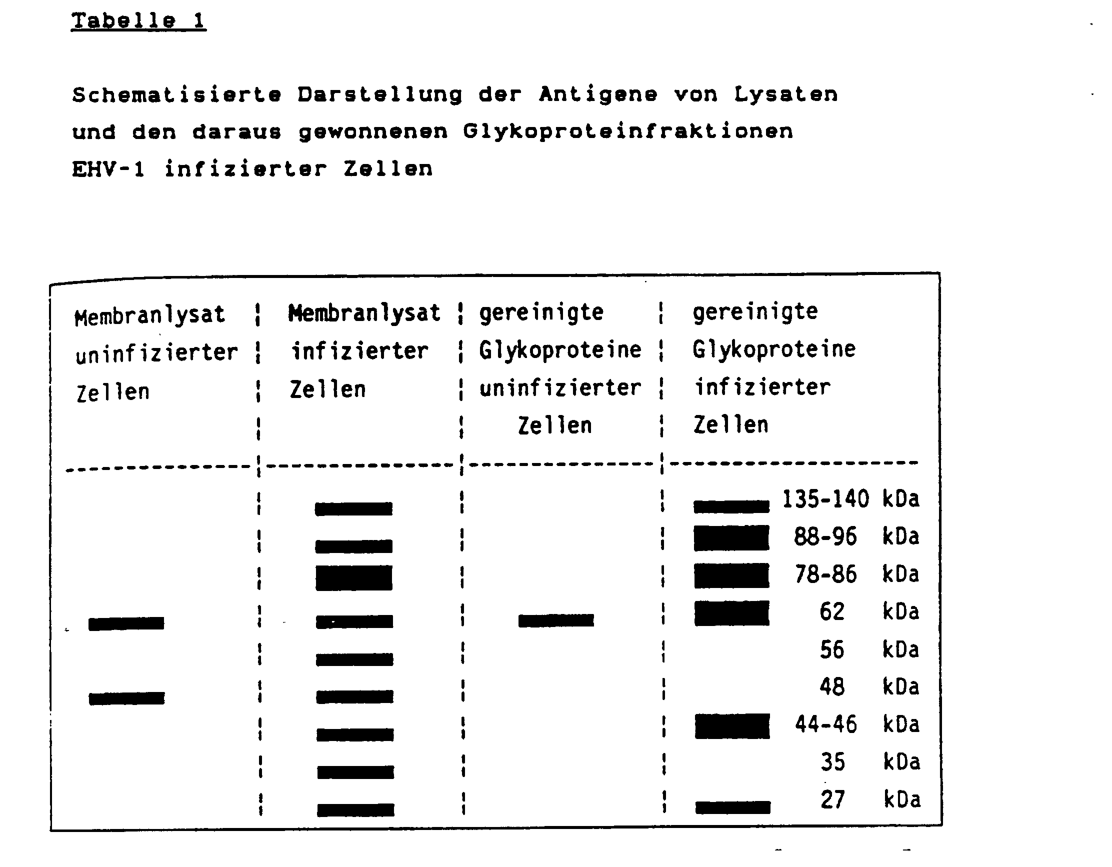

### 3. Spezifität der gereinigten Glykoproteine

Zunächst wurde analysiert, inwieweit die bisher mit dem Standardserum "Maienschein" erkannten Antigene der gereinigten Glykoproteine EHV-1 infizierter Zellen auch durch die Immunfärbung mit anderen Immunseren als Antigen erkannt werden.

Hierfür wurden jeweils Eluate aus Lysaten nicht infizierter Zellen neben den aus Lysaten EHV-1 infizierter Zellen in der reduzierenden SDS-Page aufgetrennt und im Immunoblot mit unterschiedlichen Seren getestet. Dabei wurden durch die homologe Immunfärbung mit dem Serum des Pferdes "Arfe" aus der EHV-1 Immunogenitätsstudie die gleichen Antigene wie mit dem Standardserum angefärbt. Auch das "Feldserum", Serum "Ellis" sowie die monospezifischen Ziegenseren gegen die EHV-1 Stämme DA35, DA8 und MSU erkannten prinzipiell das gleiche Antigenmuster wie das Serum "Maienschein". Im Einzelnen wurden in diesem Versuchsansatz Antigene mit relativen Molekulargewichten von 140, 118, 94 bis 88, 78, 62 sowie die schwer zu differenzierenden Antigene mit 48 und 44 kDa angefärbt. Dabei variierten die von den Immunseren erkannten Antigene der EHV-1 Eluate rein quantitativ, nicht qualitativ, in Abhängigkeit von den verwendeten Seren. Dies ist vermutlich auf die, trotz unterschiedlicher SN-Titer, gleichen Verdünnungen aller Seren im Immunoblot zurückzuführen.

Eine Ausnahme bildet das EHV-1 MSU Serum, das anstelle des 188 kDa Antigens ein Protein mit 105 kDa in der Glykoproteinfraktion erkannte. In den jeweils mitgefärbten Negativkontrollen bandeten schwach sichtbare Proteine nur bei der Färbung mit dem Serum "Maienschein" bei 62 kDa sowie mit dem Serum "Arfe" bei 62 und 48 kDa.

Zur Untersuchung der Kreuzreaktivität von Antigenen verschiedener EHV-1 Stamme wurden ED-Zellen mit sieben unterschiedlichen EHV-1 Stämmen infiziert und die jeweiligen Lysate in der Lektin-Affinitätschromatographie gereinigt. Um die Antigenmuster dieser Präparationen zu vergleichen, wurden die antigenen Glykoproteine nach Separation in der SDS-Page und dem Transfer auf Nitrozellulose im Western-Blot mit verschiedenen Seren immungefärbt. Die Ergebnisse sind in Tabelle 2 und 3 zusammengefaßt.

Die einzelnen Immunfärbungen ergaben ein für alle untersuchten EHV-1 Präparationen einheitliches Antigenmuster. Allerdings variierten auch hier die jeweils dominierenden Antigene in Abhängigkeit vom eingesetzten Serum. Während das "Feldserum" und das EHV-1 Kaninchenserum generell besonders deutlich mit dem 78 bis 86 kDa Antigen reagierten, dominierte in der Färbung mit dem "Maienschein"-Serum bei allen untersuchten Stämmen das 44 bis 46 kDa Protein. Eine fast gleichmäßige Färbung der antigenen Glykoproteine wurde mit dem Serum des Pferdes "Arte" erzielt.

Unterschiedlich reagierten die Glykoproteine der einzelnen EHV-1 Präparationen mit den Antikörpern des EHV-4 Ziegenserums. Hier wurden bei den EHV-1 Stämmen AB69 und DA35 zwei Proteine mit 60 und 42 kDa angefärbt. Bei dem EHV-1 Stamm DA 8 wurde ausschließlich das 60 kDa Protein als Antigen von dem EHV-4 Serum erkannt. Dagegen bandete bei allen sieben EHV-1 Stämmen jeweils ein Antigen bei 84 und bei 140 kDa. Letzteres wurde in diesem Immunoblot allerdings auch in der Negativkontrolle angefärbt.

In den jeweils parallel untersuchten Kontrollen des Eluates aus nicht infizierten Zellysaten wurde mit den drei Pferdeseren ein Protein mit 62 kDa gefärbt. Zusätzlich reagierte mit dem Serum "Maienschein" ein Protein von 64 kDa und mit dem Serum "Arfe" Proteine mit Molekulargewichten von 25 und 110 kDa.

Somit konnte die Präsenz der Antigene mit den Molekulargewichten 140, 96 bis 88, 86 bis 78, 46 bis 44 und 27 kDa bei allen untersuchten EHV-1 Stämmen in der Glykoproteinfraktion von Membranlysaten infizierter Zellen durch verschiedene Immunseren verifiziert werden.

**Tabelle 2**

| Übersicht der antigenen Membranglykoproteine der untersuchten EHV-1 Stämme in der Immunfärbung mit den Serum "Maienschein" | | | | | | | | |
|---|---|---|---|---|---|---|---|---|
| Mol. Gew. in kDa | uninfiziert | EHV-1 Stämme | | | | | | |
| | | a | b | c | d | e | f | g |
| 180 | | (+) | + | + | (+) | (+) | (+) | (+) |
| 140 | | (+) | (+) | (+) | (+) | (+) | (+) | (+) |
| 118 | | + | (+) | + | (+) | (+) | (+) | (+) |
| 96-88 | | + | + | + | + | + | ++ | + |
| 86-78 | | ++ | + | + | + | + | + | ++ |
| 64-62 | | + | + | + | + | + | + | + |
| 46-44 | | +++ | +++ | +++ | +++ | +++ | +++ | ++ |
| 27 | | (+) | (+) | (+) | (+) | (+) | (+) | (+) |

**Tabelle 3**

| Übersicht der antigenen Membranglykoproteine der untersuchten EHV-1 Stämme in Immunoblots mit fünf Immunseren | | | | | |
|---|---|---|---|---|---|
| Mol. Gew. in kDa | Seren | | | | |
| | Maienschein | Arfe | Feldserum | EHV-1 Serum | EHV-4 Serum |
| 180 | (+) | | | | |
| 140 | (+) | + | (+) | (+) | + |
| 118 | + | | + | (+) | |
| 105 | | (+) | | (+) | |
| 96-88 | ++ | ++ | + | + | (+) |
| 86-78 | + | + | +++ | +++ | + |
| 62 | + | + | + | (+) | (+) |
| 46-44 | +++ | ++ | ++ | ++ | (+) |
| 27 | (+) | + | (+) | | |
| (+) nur schwache Färbung, beziehungsweise nicht in allen Immunoblots nachweisbare Banden + bis +++ Intensität der Antigen-Antikörper-Reaktion | | | | | |

### 4. Kontrollierte Immunogenitätsstudien mit gereinigten Glykoproteinen EHV-1 infizierter Zellen

### 4.1 Antikörperreaktion gegen Homologes EHV-1

Die Primovakzination führte in den drei eingesetzten Dosierungen der beiden Formulierungen bei allen 24 immunisierten Pferden innerhalb von 5 Wochen post vaccinationem zur Induktion virusneutralisierender Antikörper. Die Boosterimpfung, die 5 Wochen nach Primovakzination durchgeführt wurde, resultierte in einem steilen Ansteigen der Serumantikörpertiter bis auf Werte von 1:100. Hier zeigten, bei geringgradig schwächerer Antikörperbildung nach Primovakzination, die mit den Con-A Formulierungen geimpften Pferde einen tendenziell besseren Boostereffekt als die mit Eluatformulierungen vakzinierten Pferde. In allen Gruppen, besonders aber bei den Gruppen B4 bis B6 (Con-A Vakzine), konnte 10 Wochen nach dem Boostern ein deutlicher Titerabfall festgestellt werden, der 22 Wochen post Booster wiederum bei den Gruppen B4 bis B6 Titer bedingte, die fast denen der O-Probe entsprachen.

27 Wochen nach Primovakzination wurden alle im Versuch befindlichen Pferde mit einheitlich 75 µg Glykoprotein/Dosis der Eluatformulierung revakziniert. Diese Auffrischungsimpfung hatte wiederum einen deutlichen Titeranstieg um 1 bis 2 Zweierpotenzen bei den Gruppen A1 bis A3 und 2 bis 3 Zweierpotenzen bei den Gruppen B4 bis B6 zur Folge.

Dabei lagen die Titerwerte der Gruppen A1 bis A3 mit Werten bis zu 1:170 trotzdem noch deutlich über denen der Gruppen B4 bis B6 (1:120). Die Pferde der Gruppen A1 bis A3, die mit Vakzinen der niedrigsten Antigendosen grundimmunisiert wurden, reagierten auf die Revakzination mit dem stärksten Titeranstieg.

Nach dieser Auffrischungsimpfung persistierten die Antikörper wesentlich länger; es konnten 25 Wochen post revaccinationem (entspricht ein Jahr nach Versuchsbeginn) noch Titer nachgewiesen werden, die in etwa denen der Höchstwerte post Booster entsprachen. Weitere 10 Wochen nach diesem Entnahmedatum waren die Antikörper dann soweit wieder abgebaut, daß die Titer im Bereich der nicht geimpften Kontrolltiere lagen.

Über den gesamten Beobachtungszeitraum ließ sich bei keinem der ungeimpften Kontrollpferde Serokonversion nachweisen. Darüber hinaus traten weder respiratorische Erkrankungen noch Virusaborte im Verlauf des Beobachtungsjahres im Gestüt auf. Damit ist sichergestellt, daß weder manifeste noch interkurrente EHV-Infektionen stattgefunden haben, und daß die dargestellten Antikörper die Folge der experimentellen Impfungen waren.

Tabelle 4 zeigt die Ergebnisse der serologischen Untersuchungen an den einzelnen Tieren, Tabelle 5 gibt die Gruppen-Ergebnisse als geometrisches Mittel der Individualwerte wieder.

### 4.2 Untersuchungen auf kreuzreagierende Antikörper gegen EHV-4

Die Titrationsergebnisse der Seren aus der beschriebenen EHV-1-Immunogenitätsstudie gegen EHV-4, Stamm T252 auf ED-Zellen, sind in Tabelle 6 wiedergegeben. Aufgrund zytotoxischer Wirkung der Seren in den niedrigen Verdünnungsstufen gegenüber den im SN-Test eingesetzten ED-Zellen ergaben sich klar ablesbare Titer zum Teil erst ab Serumverdünnungen von 1:8.

Die in den Pferden durch die Immunisierung mit Membranglykoproteinen EHV-1 infizierter Zellen induzierten Antikörper kreuzreagierten in der Virusneutralisation auch mit EHV-4. Zu den Entnahmezeitpunkten 5 Wochen post primovaccinationem, besonders aber 5 Wochen post Booster sowie 5 und 10 Wochen post revaccinationem, konnten EHV-4 neutralisierende Titer bis 1:20 nachgewiesen werden.

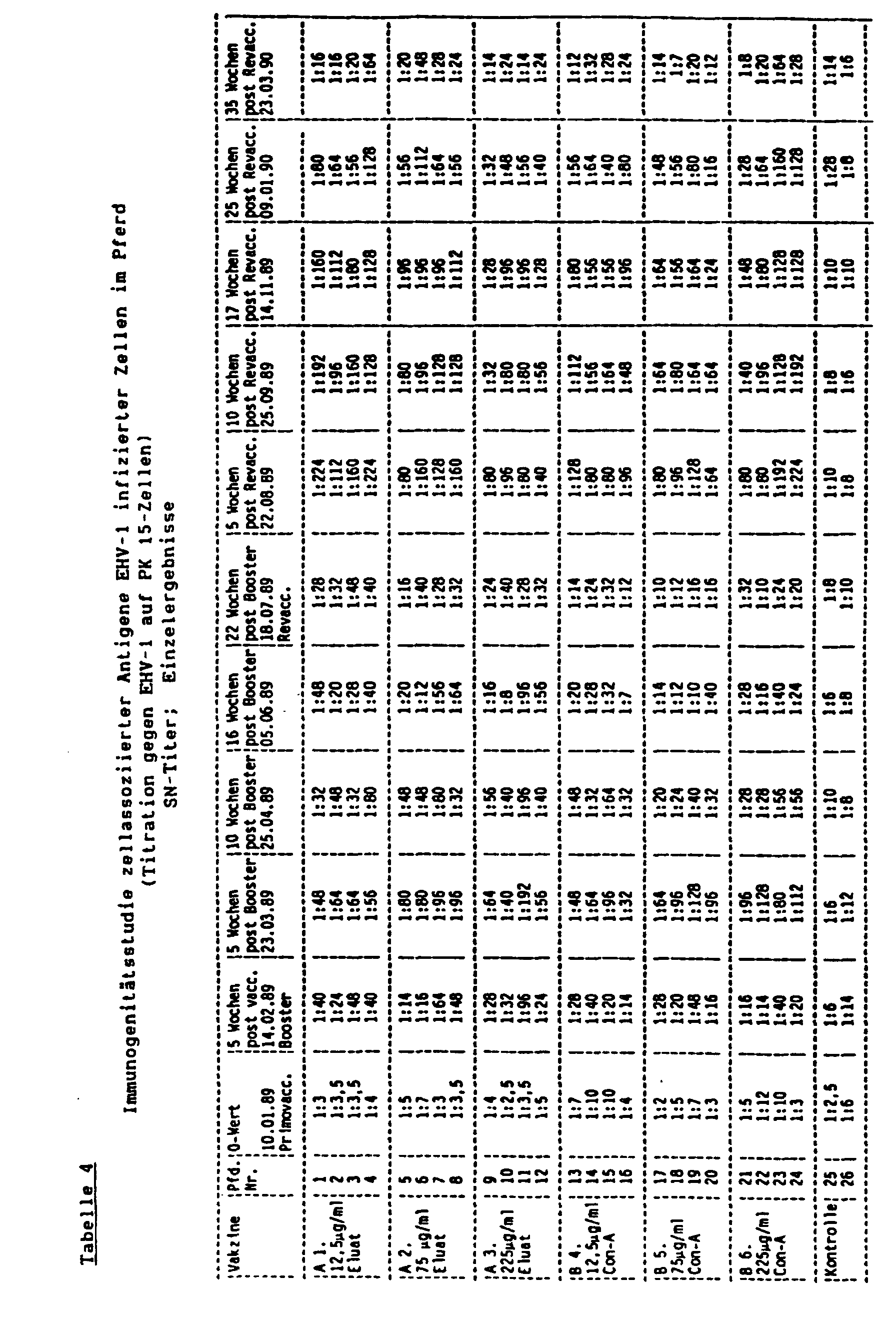

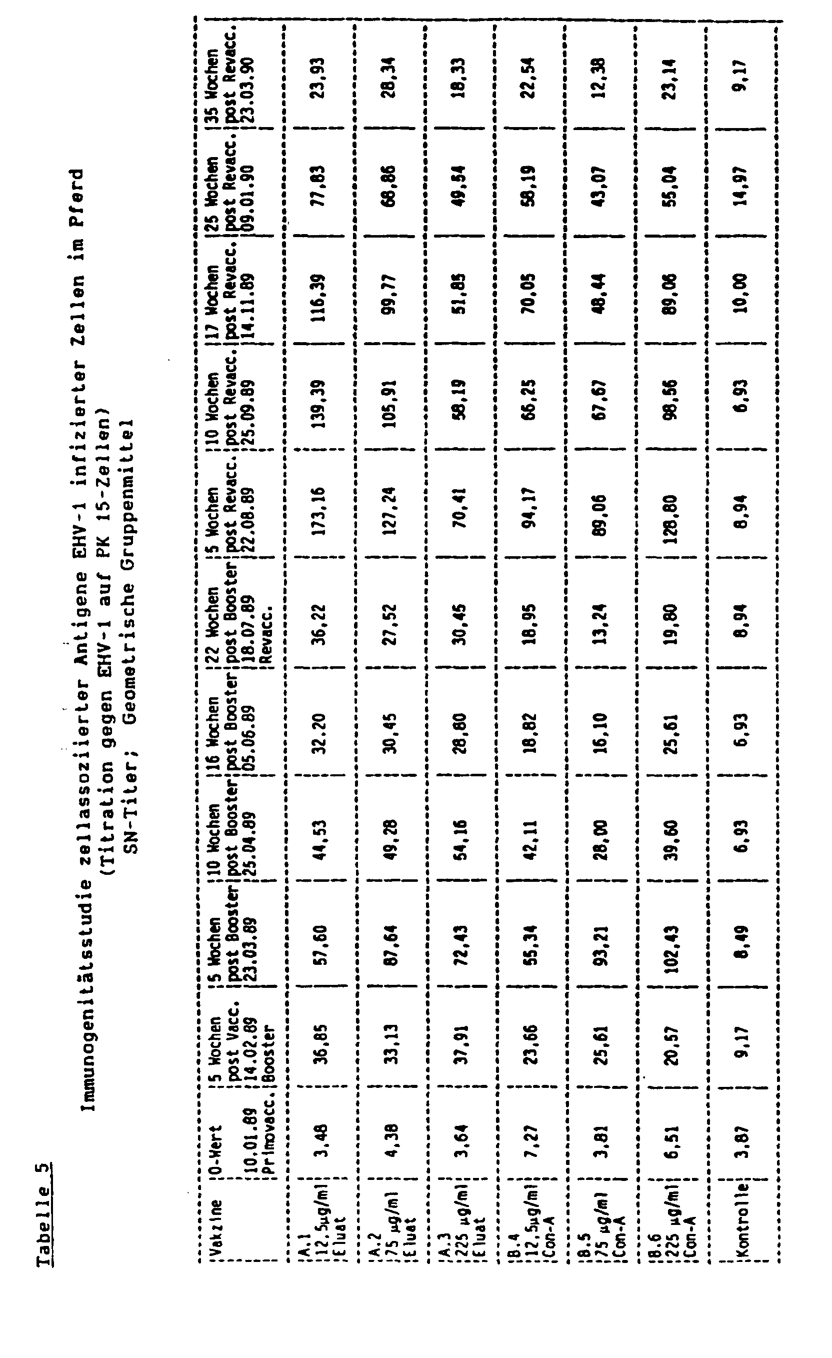

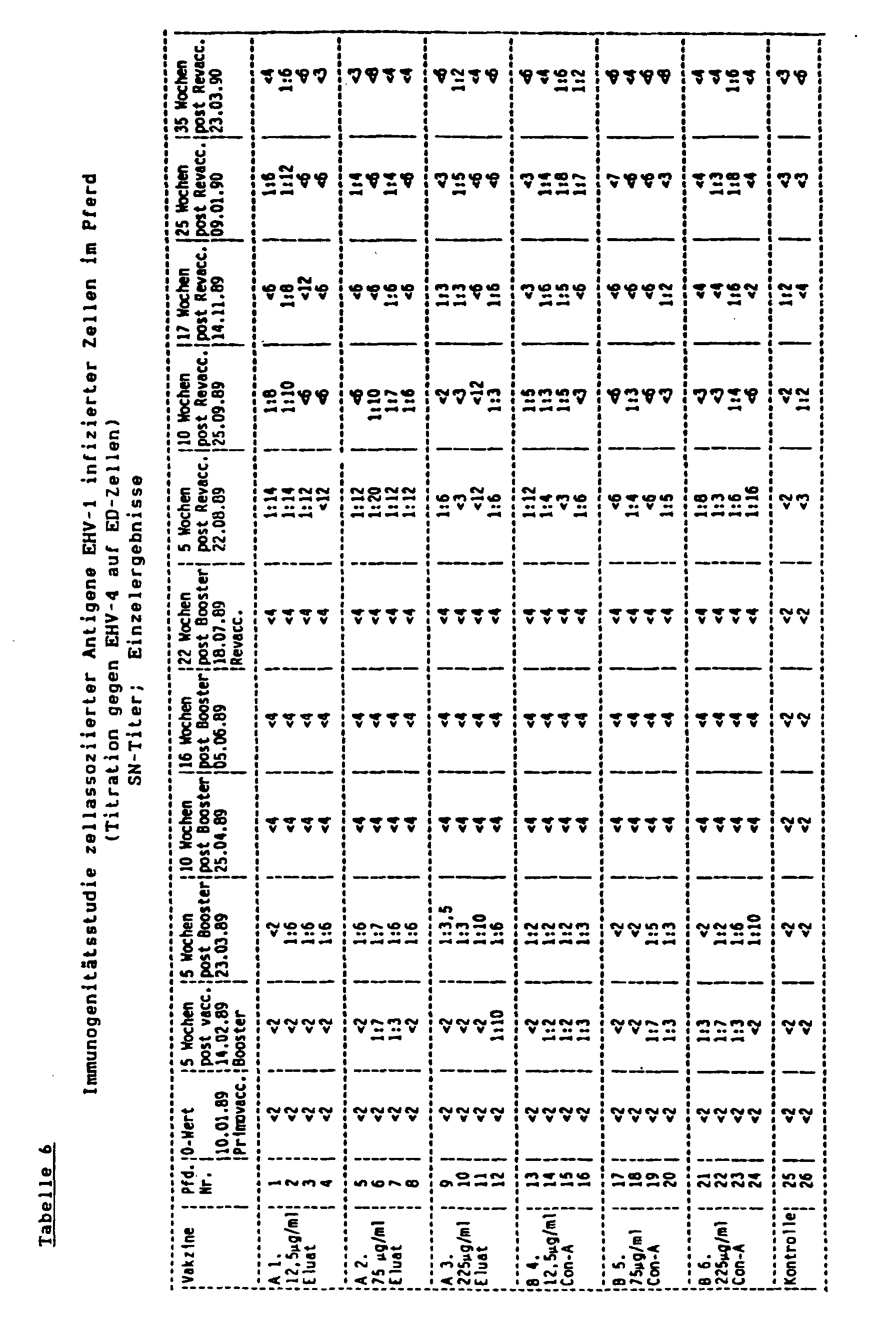

## Patentansprüche

1. Impfstoffe für Pferde gegen Infektionen mit equinen Herpesviren (EHV), dadurch erhältlich, daß man
a) equine Herpesviren vermehrt,
b) die infizierten Zellen oder das extrazelluläre Virus mit Detergentien behandelt,
c) das so erhaltene Lysat mit Lektinen behandelt,
d) den Lektin-Ligand-Komplex vom restlichen Lysat abtrennt,
e) gegebenenfalls den Lektin-Ligand-Komplex aufspaltet und das Glycoproteingemisch isoliert,
f) anschließend den Lektin-Ligand-Komplex und/oder das isolierte Glycoproteingemisch in üblicher Weise formuliert.

2. Impfstoffe gemäß Anspruch 1, **dadurch gekennzeichnet, daß** zur Komplexbildung mit den Glycoproteinen Lektine verwendet werden, die Mannose und/oder Glucose sowie deren Konjugate binden.

3. Impfstoffe gemäß Anspruch 1, **dadurch gekennzeichnet, daß** zur Komplexbildung Lektine aus Canavalia ensiformis oder Helix pomatia sowie deren Kopplungsprodukte an inerte Matrizes eingesetzt werden.

4. Impfstoffe gemäß Anspruch 1, **dadurch gekennzeichnet, daß** die Glycoproteine aus equinen Herpesviren der Serotypen 1 bis 4 isoliert werden.

5. Komplexe aus Glycoproteinen von equinen Herpesviren oder von Zellen, die mit equinen Herpesviren infiziert wurden und Lektinen sowie deren Koppelungsprodukten an inerte Matrizen.

## Claims

1. Vaccines for horses against infections with equine herpesviruses (EHV), obtainable by
a) growing equine herpes viruses,
b) treating the infected cells or the extracellular virus with detergents,
c) treating the lysate obtained in this way with lectins,
d) separating the lectin-ligand complex from remaining lysate,
e) where appropriate splitting up the lectin-ligand complex and isolating the glycoprotein mixture,
f) subsequently formulating the lectin-ligand complex and/or the isolated glycoprotein mixture in a customary manner.

2. Vaccines according to Claim 1, **characterised in that** lectins which bind mannose and/or glucose and their conjugates are used for the complex formation with the glycoproteins.

3. Vaccines according to Claim 1, **characterised in that** lectins from Canavalia ensiformis or Helix pomatia and their coupling products on inert matrices are employed for the complex formation.

4. Vaccines according to Claim 1, **characterised in that** the glycoproteins are isolated from equine herpesviruses of serotypes 1 to 4.

5. Complexes of glycoproteins of equine herpesviruses or from cells which have been infected with equine herpesviruses, and lectins and their coupling products on inert matrices.

## Revendications

1. Vaccins pour chevaux contre les infections provoquées par des herpèsvirus équins (HVE) obtenus par le fait que :
on multiplie les virus EHV,
b) on traite avec des détergents les cellules infectées ou le virus extracellulaire,
c) on traite le lysat ainsi obtenu avec des lectines,
d) on sépare le complexe lectine-ligand du lysat résiduel,
e) on dissocie le cas échéant le complexe lectine-ligand et on isole le mélange de glycoprotéines,
f) on formule ensuite de la manière habituelle le complexe lectine-ligand et/ou le mélange de glycoprotéines isolé.

2. Vaccins suivant la revendication 1, **caractérisés en ce qu'**on utilise, pour la formation de complexes avec les glycoprotéines, des lectines qui fixent le mannose et/ou le glucose ainsi que leurs conjugués.

3. Vaccins suivant la revendication 1, **caractérisés en ce qu'**on utilise pour la formation de complexes des lectines tirées de Canavalia ensiformis ou Helix pomatia ainsi que leur produits de couplage à des matrices inertes.

4. Vaccins suivant la revendication 1, **caractérisés en ce que** les glycoprotéines sont isolées d'herpèsvirus équins des types sérologiques 1 à 4.

5. Complexes de glycoprotéines d'herpèsvirus équins ou de cellules qui ont été infectées par des herpesvirus équins, et de lectines ainsi que leurs produits de couplage à des matrices inertes.
